# EUROPEAN PATENT SPECIFICATION

(11) **EP 4 051 201 B1**
(45) Date of publication and mention of the grant of the patent: **29.05.2024**
(21) Application number: 20811093.2
(22) Date of filing: 29.10.2020
(51) Int. Cl.: A61F 13/02, A61F 13/00, A61F 13/06, A61F 13/10

(54) **DECOMPRESSION THERAPY TREATMENT SYSTEM**
BEHANDLUNGSSYSTEM FÜR DEKOMPRESSIONSTHERAPIE
SYSTÈME DE TRAITEMENT DE THÉRAPIE PAR DÉCOMPRESSION

(30) Priority: 01.11.2019 US 201962929215 P; 31.12.2019 US 201962955534 P
(43) Date of publication of application: 07.09.2022
(73) Proprietor: KCI Manufacturing Unlimited Company, Dublin, D01C4E0 (IE)
(72) Inventor: JAHANIAN, Shervin, San Antonio, Texas 78265 (US); RANDOLPH, Larry Tab, San Antonio, Texas 78265 (US); SIMMONS, Tyler H., San Antonio, Texas 78265 (US); KAZALA, Richard M., San Antonio, Texas 78265 (US); REHBEIN, Jonathan G., San Antonio, Texas 78265 (US)
(74) Representative: Simmons & Simmons
(86) International application number: PCT/IB2020/060151
(87) International publication number: WO 2021/084465

(56) References cited:
- CA-A1- 3 060 484
- US-A1- 2014 276 288
- US-A1- 2015 032 035

## Description

### BACKGROUND

Swelling associated with trauma or certain pathologies (e.g. lymphedema) may cause various medical complications. For example, swelling can cause discomfort and pain, may limit range of motion, or otherwise negatively impact patient quality of life. Swelling may also limit the ability of a medical provider to medically image, view, access underlying tissue, or may otherwise interfere in the treatment of a patient, and thus may pose an impediment to the healing and recovery of the patient. In certain circumstances, swelling may lead to even more severe consequences, such as, e.g., atrophy of surrounding muscle tissue. US 2014/0276288 A1 discloses for example a system for for treating a tissue site by applying vacuum to stimulate blood flow by perfusion thereby reducing reducing swelling and inflammation, but also accelerating healing by increasing blood flow and lymph flow.

It would be advantageous to provide a durable, reusable treatment system that could be easily applied to a tissue site and which could be washed (or otherwise sterilized) between uses, and which could reliably and repeatedly be operated to provide decompression therapy to increase blood perfusion and lymphatic flow at a tissue site to reduce swelling.

### SUMMARY

The invention is defined by the claims in which there is required an apparatus for applying a lifting force to a tissue site of a patient, the apparatus comprising: an occlusive layer sealed to a patient around a tissue site to define a substantially air-tight chamber; a decompression layer disposed within the chamber defined by the occlusive layer at a location proximate the tissue site, the decompression layer comprising a compressible fabric defining one or more channels therethrough; and a connector provided along the occlusive layer, the connector configured to fluidly couple the chamber to a vacuum source; wherein, the decompression layer has a centre of stiffness located closer to a surface of the decompression layer facing away from the tissue site than to a surface of the decompression layer facing the tissue site such that upon operation of the vacuum source, the decompression layer is configured to compress in a direction away from the tissue site.

A selection of optional features is set out in the dependent claims.

### BRIEF DESCRIPTION OF THE DRAWINGS

FIG. 1 is side view of a decompression treatment system showing a partial cross-sectional view of a dressing of the decompression treatment system, according to an illustrative embodiment;
FIG. 2 is a cross-sectional view taken along line 2-2 of FIG. 1;
FIG. 3 is an exploded, perspective view of a dressing of a decompression treatment system, according to an illustrative embodiment;
FIG. 4A is a schematic representation of the collapse of a decompression layer of a dressing of a decompression treatment system, according to an illustrative example not part of the invention;
FIG. 4B is a schematic representation of the collapse of a decompression layer of a dressing of a decompression treatment system, according to an illustrative embodiment;
FIG. 5 is a perspective view of a material forming the decompression layer of a dressing of a decompression treatment system, according to an illustrative embodiment;
FIGS. 6A-6F are cross-sectional view of decompression layers, according to illustrative embodiments;
FIG. 7 is a table illustrating a comparison of the performance of the illustrative decompression layers of FIGS. 6A-6E to the performance of a reticulated foam-based decompression layer during use of a decompression treatment system according to an illustrative embodiment;
FIG. 8A is a perspective view of a dressing of a decompression treatment system, according to an illustrative embodiment;
FIG. 8B is a perspective view of the dressing of FIG. 8A being attached to a patient, according to an illustrative embodiment;
FIG. 8C is a perspective view of the dressing of FIG. 8A attached to a patient, according to an illustrative embodiment;
FIG. 9A is a perspective view of a dressing of a decompression treatment system, according to an illustrative embodiment;
FIG. 9B is a perspective view of the dressing of FIG. 9A attached to a patient, according to an illustrative embodiment;
FIG. 10 is a perspective view of a dressing of a decompression treatment system, according to an illustrative embodiment;
FIG. 11A is an exploded, perspective view of a dressing of a decompression treatment system, according to an illustrative embodiment; and
FIG. 11B is a perspective view of the dressing of FIG. 11A attached to a patient, according to an illustrative embodiment.

### DETAILED DESCRIPTION OF ILLUSTRATIVE EMBODIMENTS

Before turning to the figures, which illustrate certain exemplary embodiments in detail, it should be understood that the present disclosure is not limited to the details or methodology set forth in the description or illustrated in the figures. It should also be understood that the terminology used herein is for the purpose of description only and should not be regarded as limiting.

Referring generally to the FIGURES, a decompression therapy treatment system for applying a vacuum to intact skin extending over, or surrounding, different types of treatment tissue sites (such as, e.g., bone tissue, adipose tissue, muscle tissue, neural tissue, dermal tissue, vascular tissue, connective tissue, cartilage, tendons, ligaments, etc.) is described according to various embodiments. The application of vacuum to intact skin provided by the treatment system imparts a pulling (e.g., lifting) force to the intact skin, which decompresses the treatment tissue site and thereby increases the perfusion of blood and other fluids (e.g. lymphatic flow) at the treatment tissue site.

The decompression of the treatment tissue site resulting from the operation of the treatment system may advantageously be used to reduce swelling at a tissue site. The treatment system is configured for use in both medical and non-medical settings, and may be used to treat swelling occurring as a result of a variety of different conditions. For example, the treatment system may be used in a home setting by a patient to treat swelling resulting from an injury, over-exertion, an underlying medical condition (e.g., lymphedema), etc.

In yet other embodiments, the treatment system may also be used in a medical setting, such as, e.g., to reduce swelling during pre- and/or post-operative care of a patient. For example, reducing swelling at a treatment site (e.g., caused by a broken bone, edema, tissue sprain, tissue strain, etc.) prior to surgery may advantageously facilitate access to underlying tissue at a target surgical site, reduce surgery time and/or improve the outcome of surgical treatment. Use of a treatment system according to any of the embodiments described herein prior to surgical treatment may advantageously decrease the time needed to reduce swelling at the target surgical site to an acceptable degree of swelling as compared to the time that would be required to reduce swelling using conventional methods of treating swelling. For example, use of the treatment system may reduce swelling to an acceptable degree within 3 to 7 days of initiation of treatment using the treatment system.

In addition to the use of the treatment system to reduce swelling, the decompression therapy provided by the treatment system may advantageously also be used in the treatment of a variety of other medical conditions or ailments. As one non-limiting example, the treatment system may be used for the acute treatment of pain and/or inflammation (occurring, e.g., as a result of a sprain or other stress at a tissue site). In yet other situations, the treatment system may be used to increase blood perfusion and/or lymphatic flow at a treatment tissue site to minimize the effects of over-exertion (e.g., following athletic training or other intense activity).

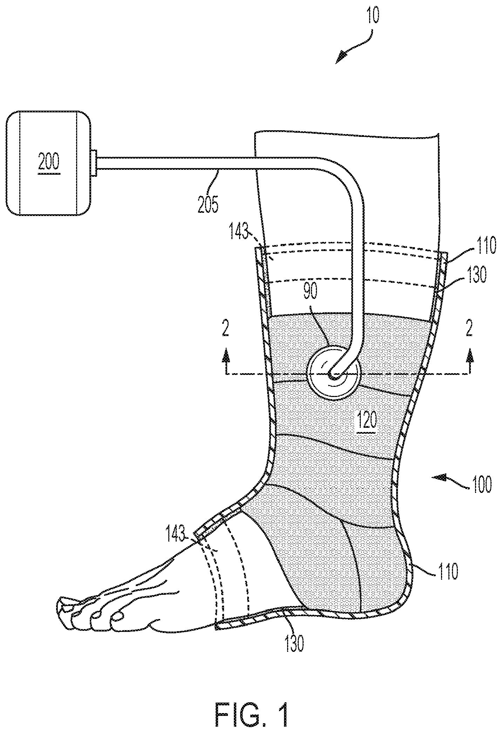

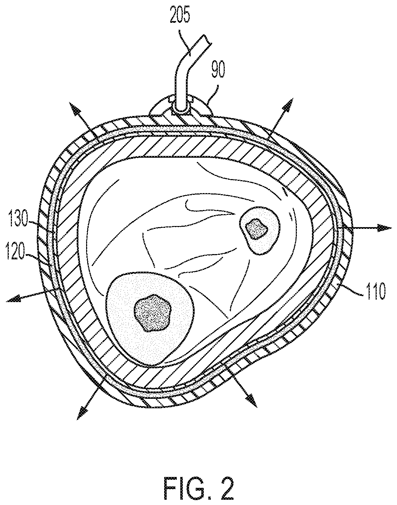

Referring to FIG. 1, the treatment system 10 generally comprises a dressing 100 configured to be attached to a patient at a location surrounding a treatment tissue site, and an air displacement device 200 (e.g., vacuum source, negative pressure pump, etc.) configured to provide a source of negative pressure to a treatment chamber defined between the dressing 100 and the treatment tissue site. Upon operation of the air displacement device 200, the treatment chamber defined between the intact skin of the patient and the dressing 100 functions as a decompression chamber in which the skin and underlying tissue is subject to an outward pulling (e.g. lifting) force (representatively illustrated by the arrows in FIG. 2) as air is evacuated from the treatment chamber. An optional controller coupled to one or both of the dressing 100 and air displacement device 200 may control the application of decompression therapy to the treatment site using the treatment system 10.

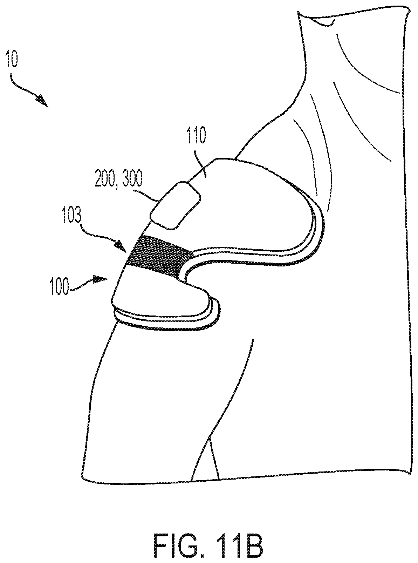

As illustrated by the treatment system 10 embodiment of FIG. 1, the dressing 100 and air displacement device 200 are optionally provided as discrete, separate components that are located remotely from one another. In such embodiments, the air displacement device 200 is fluidly and sealingly coupled to the treatment chamber via an external tubing 205. An optional connector port 90 sealingly attached around an opening 111 extending through the dressing 100 may facilitate the fluid connection between the treatment chamber and the air displacement device 200. In other embodiments (such as, e.g., embodiments in which the dressing 100 is configured to be wound around the patient) other connector port structures and/or configurations may be used to sealingly engage and fluidly couple the air displacement device 200 and the dressing 100. As shown in FIG. 11B, in yet other embodiments, the air displacement device 200 is optionally integrated into a module 300 that is fixedly, or removeably, attached to the dressing 100 to define an integral, self-contained, one-piece treatment system 10.

In addition to the use of the treatment system 10 as a standalone decompression therapy device, in various embodiments the treatment system 10 may be used in conjunction with (and may optionally be integrated into) one or more additional treatment systems. For example, although the treatment system 10 has been described as being used to impart a pulling force onto intact skin surrounding a treatment tissue site, in some embodiments, the treatment system 10 may be used to impart a pulling force onto a wound. In some such embodiments, the treatment system 10 is optionally applied atop (or integrated into) a wound dressing of a negative pressure wound therapy system ("NPWT" system). In yet other embodiments, the treatment system 10 may be used with a variety of other treatment systems, such as, e.g., a heat treatment system, systems configured to treat fractured bones, etc.

### Dressing

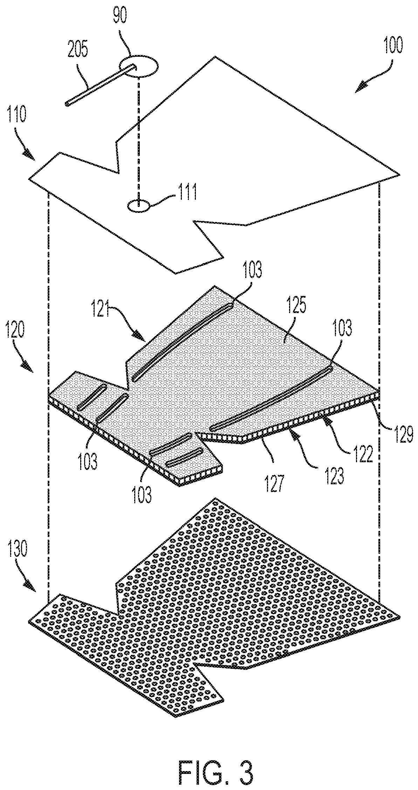

Referring to FIG. 3, the dressing 100 generally includes a flexible occlusive layer 110 and a compressible decompression layer 120 (e.g., manifolding layer, macro-mesh layer, compressible layer, collapsible layer, etc.) comprising a plurality of flow channels extending therethrough. The occlusive layer 110 is configured to be attached to a patient (e.g., using an optional seal member) to define a treatment chamber surrounding the treatment tissue site. Upon attachment of the dressing 100 to a patient, the decompression layer 120 is arranged within the treatment chamber and extends along the treatment tissue site. An optional interface layer 130 extends between the skin of the patient and the decompression layer 120.

During operation of the treatment system 10, the evacuation of air from the treatment chamber occurring upon initiation of the air displacement device 200 causes the occlusive layer 110 and decompression layer 120 to be drawn towards the intact skin surrounding the treatment tissue site. Once the vacuum applied by the air displacement device 200 has removed most of the air from the treatment chamber, the continued application of negative pressure to the treatment chamber causes the compressible decompression layer 120 to collapse (e.g., compress) in on itself. This sustained application of negative pressure to the treatment chamber and collapse of the decompression layer 120 causes the intact skin at the treatment tissue site to be pulled outwardly (such as shown by the arrows of FIG. 2), thereby stimulating blood perfusion and lymphatic flow in the subcutaneous portions of the treatment tissue site.

### A. Occlusive Layer

The occlusive layer 110 is configured to be sealed to the skin of a patient to envelop (e.g., surround, extend over, cover, etc.) the treatment tissue site. In some embodiments, such as, e.g., where the occlusive layer 110 is defined by a sleeve-like, boot-like, or other annular structure and/or by a sheet-like or tape-like structure configured to be wrapped about an anatomical structure, the occlusive layer 110 extends by approximately 360° (i.e. circumscribes) or more than 360° (i.e. the occlusive layer 110 wraps around upon itself) about a limb, extremity or other anatomical structure of the patient. In other embodiments (such as, e.g., during treatment of a knee, shoulder, elbow, etc.) the occlusive layer 110 is optionally defined by a sheet-like structure that extends less than 360° (e.g., less than 180°) around an anatomical structure of the patient.

Upon operation of the air displacement device 200, the sealed attachment between the occlusive layer 110 and the skin of the patient forms a sealed decompression treatment chamber via which negative pressure is transmitted to the treatment tissue site. An opening 111 is optionally defined through the occlusive layer 110, via which the treatment chamber is fluidly coupled to the air displacement device 200 of the treatment system 10. Alternatively, the treatment chamber is fluidly coupled to the vacuum source via a connector interposed between the skin of the patient and a lower surface of the occlusive layer 110.

The occlusive layer 110 may be formed from a variety of materials that are capable of maintaining a desired vacuum within the treatment chamber during use of the treatment system 10. The occlusive layer 110 is optionally formed from a material having a high MVTR, to allow moisture (e.g. perspiration) to be evaporated from the treatment tissue site during use of the treatment system 10. The material selected for the occlusive layer 110 is advantageously also sufficiently strong and resilient to allow the occlusive layer 110 to withstand extended periods of use of the treatment system 10. In embodiments in which the occlusive layer 110 is reusable, the material forming the occlusive layer 110 is optionally also sufficiently durable to allow the occlusive layer 110 to be cleaned (e.g. washed) between uses.

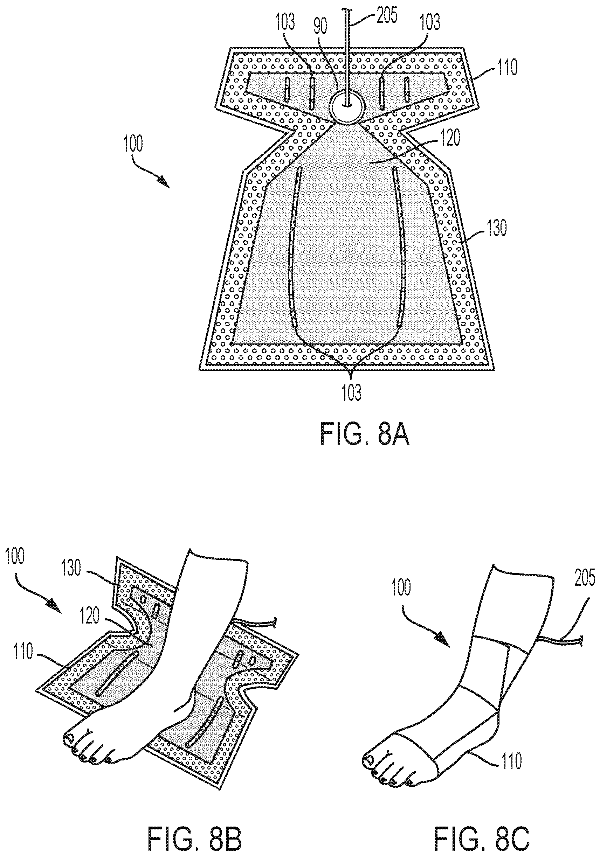

As shown in FIG. 3, in some embodiments the occlusive layer 110 is provided as a discrete and separate component of the dressing 100 which is integrated with the other components (e.g., decompression layer 120, interface layer 130, etc.) during attachment of the dressing 100 to the patient. In some such embodiments, the occlusive layer 110 optionally is provided with an adhesive along a lower surface thereof. Such a peel-and-place arrangement (in which the occlusive layer 110 is integrated with a seal member) allows the dressing 100 to be quickly wrapped around (or otherwise attached to) an anatomical structure of a patient (e.g., a foot, leg, arm, etc.), enabling a quick attachment of the dressing 100 to a patient. Referring to the representative embodiments of FIGS. 8A, 9A and 10, in other embodiments, the occlusive layer 110 is alternatively removably, or fixedly, integrated with the decompression layer 120.

Non-limiting examples of materials that may be used for the occlusive layer 110 include: polyurethane film (e.g., ESTANE 5714F), other polymer films such as, but not limited to poly alkoxyalkyl acrylates and methacrylates (e.g., such as those described in Great Britain Patent Application No. 1280631A filed November 22, 2002, the entire disclosure of which is incorporated by reference herein), laminated fabrics (e.g., polyrurethane laminated fabric, expanded polytetrafluoroethylene laminated fabric, etc.), polymer-coated fabrics, fabrics made from various synthetic fibers, etc.

### B. Decompression Layer

The decompression layer 120 (e.g., manifolding layer, macro-mesh layer, compressible layer, collapsible layer, etc.) is configured to impart a pulling, or lifting, force onto the skin at the treatment tissue site. The decompression layer 120 is formed from a material including (or defining) a plurality of flow channels (e.g. pathways, passageways, pores, etc.) therethrough. The flow channels of the decompression layer 120 allow for a sustained transmission (e.g., manifolding) of negative pressure to the treatment tissue site during operation of the treatment system 10. Some or all of the flow channels are optionally interconnected to improve the distribution of fluids (e.g. air) provided to or removed from the treatment tissue site. The decompression layer 120 is formed from a compressible material having a stiffness sufficient to provide airflow through the flow channels at negative pressures up to at least approximately 150 mmHg.

Referring to FIG. 2, as air is evacuated from the treatment chamber during operation of the treatment system 10, the greater stiffness of the skin/muscle/bone underlying the treatment tissue site relative to the stiffness of the dressing 100 causes the occlusive layer 110 to be drawn against an outwardly-facing surface 125 (e.g. an upper surface, an outer surface, a surface facing away from the tissue site, etc.) of the decompression layer 120, and also causes a tissue-facing surface 127 (e.g. a lower surface, an inner surface, etc.) of the decompression layer 120 to be drawn towards and against (directly, or indirectly via an optional interface layer 130) the skin at the treatment tissue site. Once air has substantially been evacuated from the treatment chamber, the continued application of a vacuum to the treatment chamber results in the collapse of the compressible decompression layer 120.

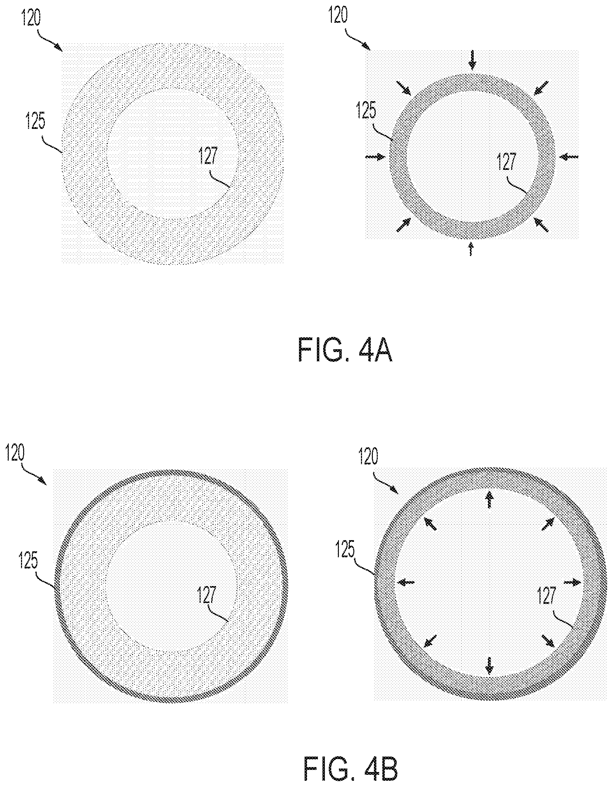

The direction (i.e. outward/inwards; upwards/downwards; away from/towards the tissue site; in a radial direction; in a vertical direction; etc.) in which the decompression layer 120 collapses (e.g., compresses) varies depending on the construction of the decompression layer 120. The collapse of a decompression layer 120 comprising a single layer, and formed from a material having a uniform density, in response to a vacuum is representatively illustrated in FIG. 4A. As shown by the arrows of FIG. 4A, such a decompression layer 120 having a center of stiffness that is located closer to the tissue-facing surface 127 than the outwardly-facing surface 125 of the decompression layer 120 will compress such that the outwardly-facing surface 125 of the decompression layer 120 is drawn towards the tissue-facing surface 127 of the decompression layer 120.

The collapse of a decompression layer 120 comprising an outer portion (i.e. a portion of the decompression layer 120 adjacent the outwardly-facing surface 125 that faces away from the tissue site) formed from a stiff material and an inner portion (i.e. a portion of the decompression layer 120 adjacent the tissue-facing surface 127) formed from a softer material in response to a vacuum is representatively illustrated in FIG. 4B. In contrast to the representative decompression layer 120 illustrated in FIG. 4A, a decompression layer 120 such as illustrated in FIG. 4B, which is defined by a center of stiffness that is located closer to the outwardly-facing surface 125 than the tissue-facing surface 127 of the decompression layer 120, will experience a parallel plate effect upon being subject to a vacuum during use of the treatment system 10. As illustrated by the arrows of FIG. 4B, this sandwiched arrangement of a softer, inner-half portion of the decompression layer 120 between two stiffer structures (i.e., the skin/muscle/bone underlying the treatment tissue site and a relatively harder, outer-half portion of the decompression layer 120) results in the tissue-facing surface 127 of the decompression layer 120 being drawn towards the outwardly-facing surface 125 of the decompression layer 120 as the decompression layer 120 collapses. The pulling force imparted onto the skin at the treatment tissue site as a result of such an outwardly directed collapse of the decompression layer 120 is effective for enhancing lymphatic flow and blood perfusion at the treatment tissue site.

Given the impact of the parallel plate effect on lymphatic flow and blood perfusion at a tissue site, the decompression layer 120 is advantageously constructed such that the center of stiffness of the decompression layer 120 is located closer to the outwardly-facing surface 125 than the tissue-facing surface 127 of the decompression layer 120). The decompression layer 120 is also advantageously constructed from a material that is sufficiently flexible to allow the decompression layer 120 to be secured to a patient and to allow for a range of motion of the body part to which the dressing 100 is attached during use of the treatment system 10.

The decompression layer 120 is advantageously also formed having sufficient structural integrity and resilience to withstand repeated applications of negative pressure thereto over the course of operation of the treatment system 10 (e.g., for periods of up to, or greater than, one week). To facilitate the reuse of the treatment system 10 with the same, or other, patients, the decompression layer 120 is additionally optionally constructed having a durability that allows the decompression layer 120 to be washed in-between uses.

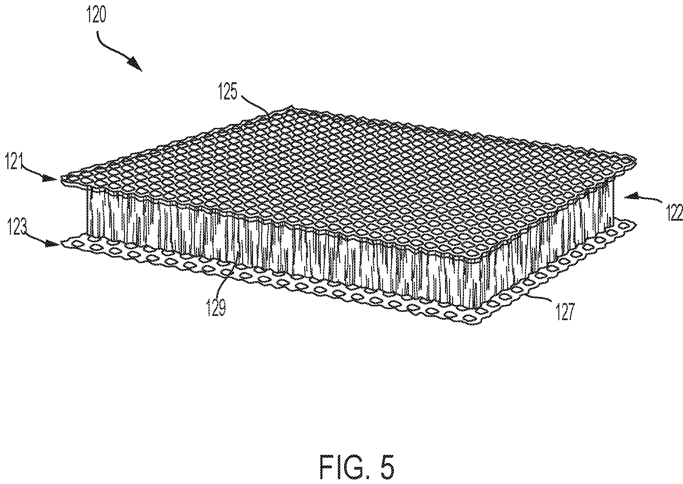

Referring to FIG. 5, a flexible, resilient and durable decompression layer 120 construction configured to impart an increased pulling force on skin at a tissue treatment site is shown according to an exemplary embodiment. In the decompression layer 120 embodiment of FIG. 5, the decompression layer 120 is defined by a macro-mesh material comprising a lower layer 123, an upper layer 121, and an intermediate layer 122. Upon integration of the decompression layer 120 into the tissue treatment system 10, a lower surface of the lower layer 123 defines the tissue-facing surface 127 (e.g., a lower surface, an inner surface, a radially inwardly extending surface, etc.) of the decompression layer 120, and an upper surface of the upper layer 121 defines the outwardly-facing surface 125 (e.g. an upper surface, an outer surface, a radially outwardly extending surface, etc.) of the decompression layer 120. The upper layer 121 and lower layer 123 are vertically offset from one another, as well as interconnected to one another, via the intermediate layer 122 (e.g., a connector layer).

The upper layer 121 and lower layer 123 defining the macro-mesh material forming the decompression layer 120 may be defined by a variety of different materials. To provide the decompression layer 120 with a desired degree of resilience and durability, one or both of the upper layer 121 and the lower layer 123 are formed from a textile material. The textile may be defined by a variety of different woven or non-woven patterns, weights, densities, fibers, stiffnesses, etc., depending on the desired properties of the decompression layer 120. According to various embodiments, one or both of the upper layer 121 and the lower layer 123 are formed from a polymer or nylon material (e.g., a polymer or nylon mesh).

To provide the decompression layer 120 with the desired offset center of stiffness (i.e., a center of stiffness that is located closer to the outwardly-facing surface 125 of the decompression layer 120), the upper layer 121 is formed from a different material, has a different construction, or otherwise varies from the lower layer 123. For example, the upper layer 121 is formed from a material having a greater stiffness than the material used for the lower layer 123. The materials selected for the upper layer 121 and/or lower layer 123 may optionally include a coating (e.g., an anti-microbial coating, a hydrophobic coating, etc.), to provide the decompression layer 120 with additional desired features.

The intermediate layer 122 may be formed from a variety of different materials. As shown in FIG. 5, according to various embodiments, the intermediate layer 122 is formed of a plurality of durable, resilient, and flexible (e.g., collapsible, deflectable, bendable, compressible, etc.) filament fibers 129 that allow the decompression layer 120 to collapse (e.g., compress, or otherwise cause a distance between the upper layer 121 and lower layer 123 to be reduced) one or more times during use of the treatment system 10. The filament fibers 129 forming the intermediate layer 122 may be defined by a variety of yarn types (e.g. monofilament, multifilament, spun, etc.), diameters, lengths, materials, weights, denier, densities, stiffness, etc. The selection and arrangement of filament fibers 129 may be varied based on the desired characteristics of the manifold layer. For example, the length and density of the filament fibers 129 forming the intermediate layer 122 may be varied based on a desired stiffness of the decompression layer 120.

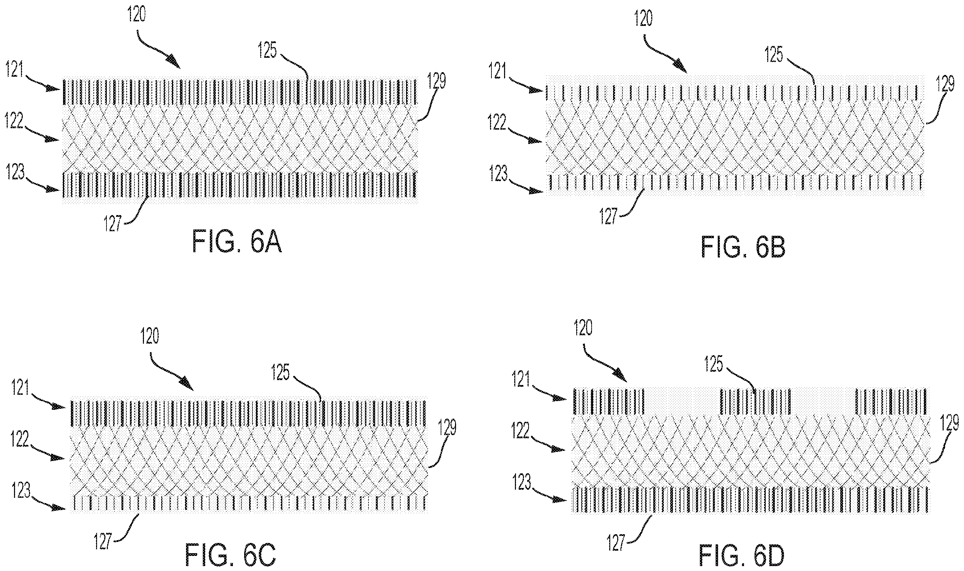

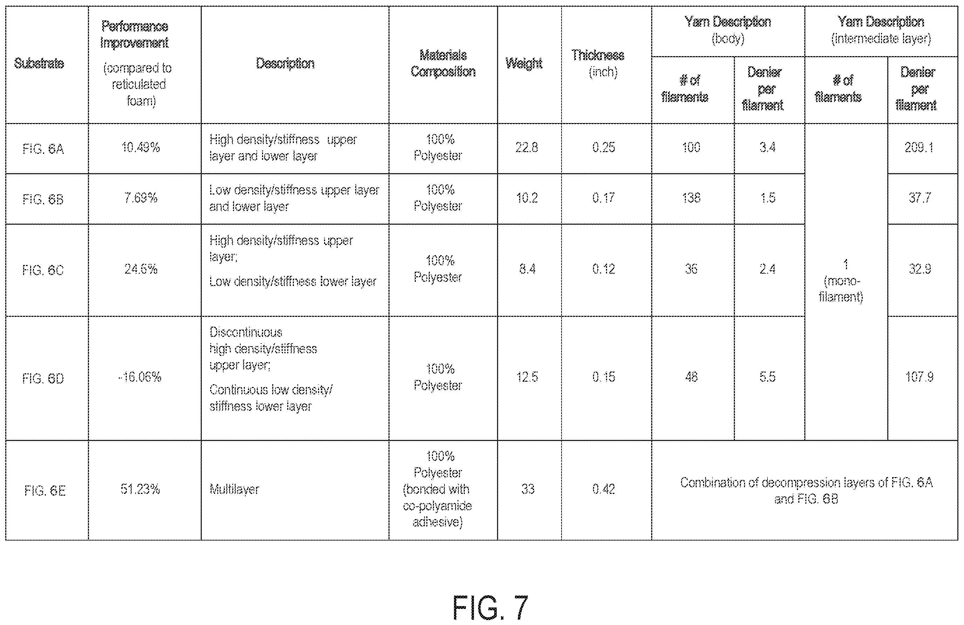

The effects of varying various properties of the dual-layered decompression layer 120 arrangement of FIG. 5 on the degree of pulling force imparted onto the skin during operation of the treatment system 10 are described with reference to FIGS. 6A-6F and FIG. 7. Non-limiting properties of the decompression layer 120 embodiments illustrated in FIGS. 6A-6E are provided in the table of FIG. 7.

In general, a decompression layer 120 comprising a macro-mesh configuration such as, e.g., representatively illustrated by the embodiment of FIG. 5, is defined by a greater stiffness than a decompression layer 120 formed of single layer, uniformly dense, reticulated foam material. Accordingly, as illustrated by the table of FIG. 7-even if the dual-layered decompression layer 120 is defined by a center of stiffness located at (or substantially at) a center of the decompression layer 120 (such as, e.g., illustrated by the embodiments of FIGS. 6A and 6B)-the structure of the dual-layer decompression layer 120 provides enhanced blood perfusion and lymphatic flow at a treatment tissue site as compared to a decompression layer formed of a single layer, uniformly dense, reticulated foam material.

For example, as shown in the table of FIG. 7, in one embodiment, a decompression layer 120 embodiment such as shown in FIG. 6A which comprises a macro-mesh configuration having a high-density and/or high-stiffness (e.g., formed of a polyester material having a denier of approximately 3.4) upper layer 121 and lower layer 123, may increase the degree of perfusion and flow at the treatment tissue site by approximately 10.5% as compared to a decompression layer formed of a single layer, uniformly dense, reticulated foam material. As also shown in the table of FIG. 7, in one embodiment, a decompression layer 120 embodiment such as shown in FIG. 6B which comprises a macro-mesh configuration having a low-density and/or low-stiffness (e.g., formed of a polyester material having a denier of approximately 1.5) upper layer 121 and lower layer 123, may increase the degree of perfusion and flow at the treatment tissue site by approximately 7.7% as compared to a decompression layer formed of a single layer, uniformly dense, reticulated foam material.

As illustrated by a comparison of the performance of the decompression layer 120 examples of FIGS. 6A and 6B as summarized in the table of FIG. 7, increasing the density (and stiffness) of the material used to form the substantially similar upper layer 121 and lower layer 123 of a decompression layer 120 such as e.g., illustrated by the embodiment of FIG. 6A will provide an increased pulling force as compared to a dual-layer decompression layer 120 embodiment having an upper layer 121 and lower layer 123 each formed from a lower density (and lower stiffness) material (such as, e.g., representatively illustrated by the embodiment of FIG. 6B).

As illustrated by the table of FIG. 7, a dual-layer decompression layer 120 arrangement, such as representatively shown in FIG. 6C comprising an upper layer 121 formed from a higher density (and higher stiffness) material and a lower layer 123 formed from a lower density (and lower stiffness) material-which accordingly is defined by a center of stiffness located closer to the outwardly-facing surface 125 of the decompression layer 120-will impart an increased pulling force on the treatment tissue site as compared to a decompression layer 120 formed having both an upper layer 121 and a lower layer 123 formed from materials having the same density (and same stiffness), such as, e.g., the decompression layer embodiments of FIGS. 6A and 6B. For example, as compared to the 10.5% improvement over foam of the decompression layer 120 embodiment of FIG. 6A (which is formed from high density/stiffness upper layer 121 and lower layer 123) and the 7.7% improvement over foam of the decompression layer 120 embodiment of FIG. 6B (which is formed from low density/stiffness upper layer 121 and lower layer 123), a decompression layer embodiment having a high density and/or high stiffness upper layer 121 and a low density and/or low stiffness lower layer 123 (such as, e.g., the embodiment of FIG. 6C) exhibits an improvement of 24.6% in perfusion and flow as compared to a decompression layer formed of a single layer, uniformly dense, reticulated foam material.

As illustrated by FIG. 7, the decompression layer 120 embodiment of FIG. 6D is formed from a high density (and high stiffness) material, and the lower layer 123 of the decompression layer 120 is also formed from a low density (and lower stiffness) material, similar to the materials used for the lower layer 123 and upper layer 121 of the decompression layer 120 embodiment of FIG. 6C. However, whereas the decompression layer 120 embodiment of FIG. 6C comprises a continuously extending upper layer 121, the upper layer 121 of the decompression layer 120 embodiment of FIG. 6D is instead defined by strips of the high density (and high stiffness) material that are separated from one another by portions of the intermediate layer 122 along which no upper layer 121 extends.

As a result of the interrupted upper layer 121 configuration of the decompression layer 120 embodiment of FIG. 6D, the center of stiffness of the decompression layer 120 of FIG. 6D is located closer to the tissue-facing surface 127 than to the outwardly-facing surface 125 of the decompression layer 120. As shown in FIG. 7, the effect of the center of stiffness of the decompression layer 120 of FIG. 6D being located closer to the tissue-facing surface 127 of the decompression layer 120 is that the decompression layer 120 embodiment of FIG. 6D imparts even less of a pulling force onto skin at the treatment tissue site than a uniformly dense, single layer reticulated foam-based decompression layer. Accordingly, as shown by the table of FIG. 7, a decompression layer arrangement such as that of FIG. 6D may result in a decrease in perfusion and flow at the treatment tissue site of 16.1% as compared to a decompression layer formed of a single layer, uniformly dense, reticulated foam material.

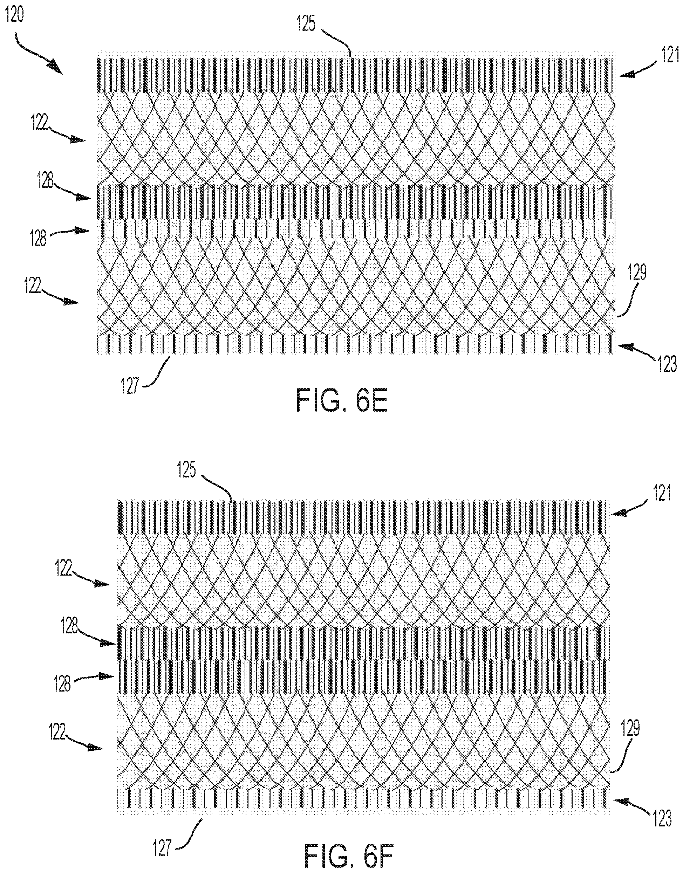

The degree of pulling force imparted onto the skin by the decompression layer 120 may be further augmented by constructing the decompression layer 120 to maximize the distance of the center of stiffness from the tissue-facing surface 127 of the decompression layer 120. As described with reference to FIG. 6C, one such option for maximizing this distance is to increase the stiffness of the upper layer 121 of the decompression layer 120 relative to the stiffness of the lower layer 123 of the decompression layer 120. As shown in FIG. 7, and illustrated by the embodiments of FIGS. 6E and 6F, an additional option for increasing the pulling force imparted onto skin during use of the treatment system 10 is to increase a thickness (i.e., distance between the outwardly-facing surface 125 and the tissue-facing surface 127) of the decompression layer 120.

As representatively illustrated by the embodiments of FIG. 6E and 6F, an increased thickness of the decompression layer 120 may be accomplished by incorporating one or more additional textile layers 128 (similar to the upper layer 121 or the lower layer 123) into the decompression layer 120 structure. As shown in FIGS. 6E and 6F, these additional one or more layers 128 may be integrated into the decompression layer 120 structure via one or more additional intermediate layers 122. To maximize the pulling force imparted by the decompression layer 120 onto the skin, the additional textile layer 128 are advantageously integrated into the decompression layer 120 in a manner that maintains the center of stiffness of the decompression layer 120 closer to the outwardly-facing surface 125. For example, as illustrated by the embodiment of FIG. 6E, the decompression layer 120 may comprise the decompression layer 120 of FIG. 6A bonded, or otherwise attached) along the outwardly-facing surface 125 of the decompression layer 120 of FIG. 6B. As shown in Table 7, such a multilayer decompression layer 120 configuration as illustrated by the embodiment of FIG. 6E may provide an increase in perfusion and flow at a treatment tissue site of 51.2% as compared to a decompression layer formed of a single layer, uniformly dense, reticulated foam material.

### C. Interface Layer

An optional interface layer 130 (i.e. skin contact layer) is disposed adjacent the skin of the patient upon the attachment of the dressing 100 to the patient. The interface layer 130 may be incorporated into the dressing 100 for a variety of reasons, and may be defined by a variety of different features. For example, the interface layer 130 may be configured to: decrease discomfort and irritation during use of the treatment system 10; provide cooling; wick liquid away from the skin; function as an antimicrobial barrier; create friction between the decompression layer 120 and the skin to enhance the lifting force imparted onto the skin by the decompression layer 120, etc.

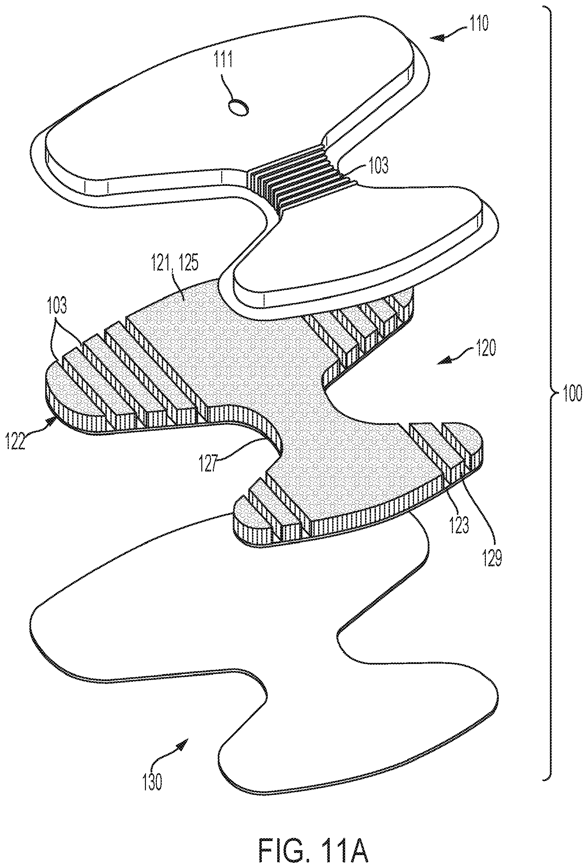

The materials forming the interface layer 130 may be selected based on the desired features of the interface layer 130. In general, the optional interface layer 130 is constructed from a light-weight, thin material that does not impede flow between the skin and the decompression layer 120, and which does not irritate the skin. As shown in FIG. 11A, in some embodiments, the interface layer 130 may comprise a textile, or other porous material, such as, e.g., a non-woven, breathable fabric. As shown in FIG. 3, in other embodiments, the interface layer 130 may be formed from an occlusive material including a plurality of perforation or holes formed therethrough. The interface layer 130 is optionally also formed having a durability and resilience sufficient to allow for reuse of the interface layer 130.

The interface layer 130 may be integrated into the dressing 100 according to a variety of arrangements. In some embodiments the interface layer 130 is provided entirely separate and detached from the decompression layer 120. In some such embodiments, the interface layer 130 may be provided as a sock or sleeve that is slid onto and around a treatment tissue site (e.g., a leg or arm of the patient). Once positioned in the desired location, the decompression layer 120 and occlusive layer 110 components of the dressing 100 are attached to the patient. Such a decoupled arrangement may advantageously allow a user to verify that the interface layer 130 lies taut and smoothly along the skin prior to attaching the remaining components of the dressing 100, thus minimizing the risk of pinching resulting from wrinkling along the interface layer 130 during use of the treatment system 10.

Alternatively, the interface layer 130 is partially, or entirely, attached along the tissue-facing surface 127 of the decompression layer 120, such as, e.g., illustrated by the embodiment of FIG. 8A. In some embodiments, the interface layer 130 is removably attached to the decompression layer 120, allowing the interface layer 130 to be detached as desired (e.g., to wash the interface layer 130 prior to reuse of the treatment system 10). In other embodiments, the interface layer 130 is instead be fixedly secured to the entirety of, or a portion of (e.g., the peripheral edges of) a lower surface of the decompression layer 120 (e.g., by heat bonding, via adhesive, via ultrasonic welding, etc.). Such a fixed attachment of the interface layer 130 and the decompression layer 120 may advantageously minimize the presence of loose spots between the interface layer 130 and decompression layer 120, which may reduce the occurrence of ridges and bubbling, and thereby minimize the risk of pinching during operation of the treatment system 10.

### D. Seal Member

A seal member of the dressing 100 is used to provide a sealing (e.g., fluid-tight) attachment between the occlusive layer 110 and an underlying surface (e.g., skin, a section of the occlusive layer 110 that has been wrapped around the patient, an optional interface layer 130, etc.) that enables a vacuum to be created and maintained within the treatment chamber surrounding the tissue treatment site. Advantageously, the seal member is structured to be sufficiently robust to continuously, or intermittently, maintain a desired negative pressure within the treatment chamber over the duration of use of the treatment system 10. The seal member is advantageously self-adhering and is able to provide a fluidtight attachment to a variety of different surfaces, including, e.g., skin, the optionally included interface layer 130, the decompression layer 120, the occlusive layer 110, etc. In embodiments in which the seal member is reusable, the seal member is advantageously sterilizeable. Alternatively, the seal member may be replaceable (e.g., removable), such that a new seal member may be used with each subsequent use of the treatment system 10.

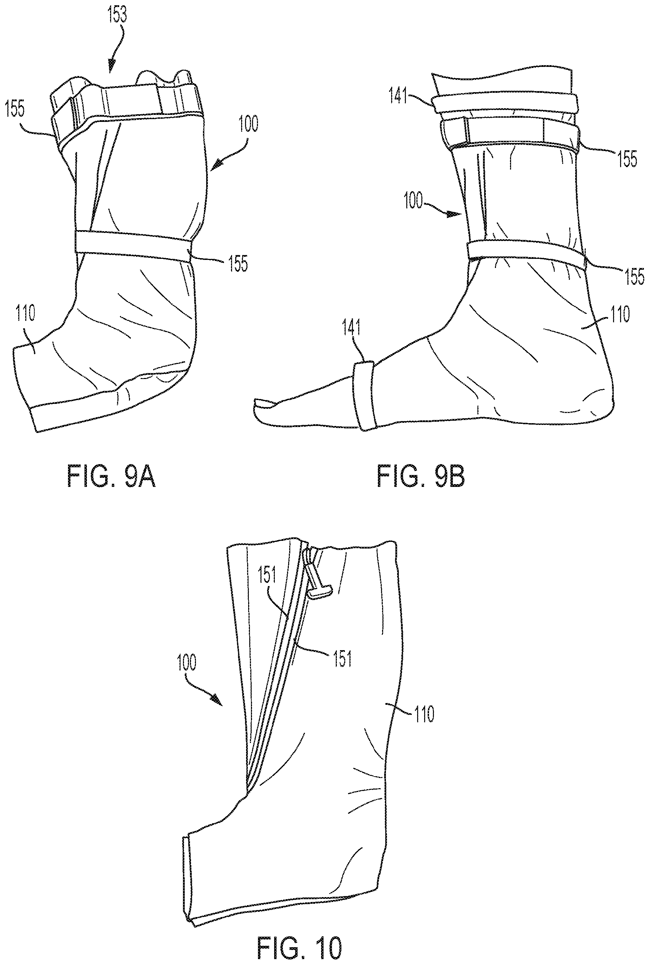

The seal member may be defined by a variety of, and combination of, various sealing structures. As shown in FIG. 9B, the seal member may optionally comprise a discrete component(s) provided separately from the other components of the dressing 100. For example, the seal member may comprise a tape-like or film-like structure 141 (e.g., thermoplastic elastomer gel strips, silicone/acrylic trilaminate film, etc.) that is applied along the entirety of, or along the outer periphery of, the upper layer of the occlusive layer 110 to secure the dressing 100 to the patient. In other embodiments, the seal member alternatively, or additionally, includes a wiper seal 143 (see, e.g., FIG. 1), adhesive (e.g., an acrylic or silicone adhesive), or other sealing structure (e.g., a gasket) that is provided along (e.g., integrated with) or disposed between, an entirety or periphery of a lower surface of the occlusive layer 110.

In various embodiments, the sealing attachment provided by the seal member may be reinforced and/or concealed by a hook-and-pile fastener, cohesive bandage, cast protector, or other structure that is positioned atop the dressing 100 following the attachment of the dressing 100 to a patient.

### Dressing Configurations

The size, shape and configuration of the dressing 100 may vary depending on a variety of factors, including, e.g., the treatment tissue site being treated, the patient being treated, the duration of the treatment being provided, etc. Additional features of the dressing 100 that may be varied depending on the desired use of the treatment system 10 include, e.g., the degree of tailoring of the dressing 100 to a particular treatment site, the extent to which the dressing 100 is attached to a patient, the incorporation of features facilitating the attachment of the dressing 100 to a patient, the degree of integration of the components of the dressing 100, etc.

As illustrated in FIGS. 9A and 10, according to various embodiments the dressing 100 defines a closed annular structure configured to extend circumferentially about an entirety of a limb or other extremity by at least 360°. Upon attachment, the dressing 100 partially, or entirely, envelops the extremity.

In some embodiments, the annular dressing 100 is defined by a sleeve-like structure having a generally tubular shape that extends between a first open end and a second open end. In other embodiments the sleeve-like annular dressing 100 extends between a first open end and a second open end, and is shaped, sized and contoured for attachment around a specific extremity of a patient. For example, referring to FIGS. 9A and 10, in some embodiments the dressing 100 defines an open-toe boot structure configured to receive a foot. In other embodiments, the annular dressing 100 defines a receiving portion that is configured to receive a portion (or the entirety) of an extremity of a user (e.g., a hand, a foot, a stump, etc.). The receiving portion is accessible via a single open end defined by the annular dressing 100. The receiving portion defined by the annular dressing 100 may be generally cylindrical in shape, or may optionally define a structure that is shape, size and contoured to receive a specific extremity of a patient. For example, the dressing 100 may comprise a glove or mitten structure for receiving a hand of a patient, or may comprise a sock, or closed-toe boot structure for receiving a foot of a patient. Such customization of the dressing 100 to a specific treatment tissue site may advantageously facilitate an air-tight attachment of the dressing 100 to the treatment tissue site. The annularly extending dressing 100 optionally includes folds and/or other articulation features configured to allow for at least a partial degree of flexion or movement during treatment using the therapy system.

In embodiments in which the dressing 100 is defined by an annularly extending structure having one open end or two open ends, and which is configured to encircle or otherwise circumscribe a portion of the patient, the dressing 100 may be formed from materials that allow the dressing 100 to stretch during application of the dressing 100 around a patient. Alternatively, or additionally, the dressing 100 optionally includes one or more features configured to facilitate the application of the dressing 100 around the patient. For example, as illustrated in FIG. 10, the dressing 100 optionally includes a slit extending partially, or entirely, along a length thereof. Mating engagement elements 151 (e.g., mating zipper teeth, hook and pile, etc.) are optionally provided along the length of each edge defining the slit to allow the edges to be selectively coupled and decoupled from one another. In some such embodiments, one or both of the edges optionally comprises a plurality of similar or identical engagement elements that are spaced generally parallel to each other at one or more locations spaced inwardly from the edge, thus allowing the diameter of the dressing 100 to be adjusted as needed.

As an alternative to (or in addition to) constricting the dressing 100 from materials that provide for a degree of stretch and/or the inclusion of a slit such as representatively illustrated in FIGS. 9A and 9B, the dressing 100 in some embodiments includes a gusset 153 that may be let out (or taken in) via the tightening of an annularly extending strap 155 (e.g., mating hook-and-pile straps, etc.) to increase (or decrease) the dimensions of the opening of the dressing 100. In yet other embodiment, the components of an annularly-shaped dressing 100 are optionally also (or alternatively) formed from elastic materials that allow the dressing 100 to stretch and expand to facilitate the insertion of a limb or other extremity into the opening of the dressing 100 during the attachment of the dressing 100 to a patient.

According to other embodiments, the dressing 100 may alternatively be defined by a flexible, sheet-like structure. The sheet-like dressing 100 may be provided in a range of shapes and sizes. As representatively illustrated by the embodiment of FIG. 3, in some embodiments the sheet-like dressing 100 is optionally shaped and sized for application to a particular treatment site.

As illustrated in FIGS. 8A and 8B, in some embodiments, a sheet-like dressing 100 may be wrapped around a treatment tissue site to substantially (e.g. entirely) circumscribe a portion of the patient (e.g. a calf, a wrist, an ankle, etc.). For example, the sheet-like dressing 100 may be configured to be wrapped around a limb or extremity of a patient by approximately 360° or more. Alternatively, as shown in FIGS. 11A and 11B, in other embodiments, the sheet-like structure defining the dressing 100 is attached to a patient as a patch, in which the outer periphery of the sheet-like dressing 100 circumscribes the treatment tissue site, but does not circumscribe the limb or extremity of the patient along with the treatment tissue site is located (e.g., the dressing 100 may extend less than 360° around a limb or extremity, such as e.g., when the dressing 100 is applied over a knee or shoulder). In some such embodiments, (and/or in other dressing 100 embodiments), the dressing 100 is optionally provided with a thin, semi-rigid, pliable (e.g. bendable, shapeable, etc.) reinforcement layer that allows the sheet-like dressing 100 to be adapted to match the contours of the treatment tissue site to which the dressing 100 is to be attached, thereby facilitating the attachment of the dressing 100 to the patient. As shown in FIGS. 11A and 11B, the sheet-like dressing 100 optionally includes folds and/or other articulation features 103 configured to allow for at least a partial degree of flexion or movement during treatment using the therapy system.

According to yet other embodiments, the dressing 100 may be provided as a flexible tape that can be wound around a treatment tissue site, or which may be attached as one or more strips atop a treatment tissue site. Such a tape-like dressing 100 arrangement may provide a user with the ability to customize the attachment of the treatment system 10 to a variety of different treatment sites and to a variety of different patients. In some embodiments, an adhesive is optionally provided along an outer periphery of the tape-like structure to facilitate the attachment of the dressing 100 to the patient. In such embodiments, the application of the tape-like structure such that adjacent segments of the tape (e.g. adjacent winding or adjacent strips) overlap may allow the dressing 100 to be attached to a patient without requiring any additional sealing of the dressing 100 to the patient. Alternatively, an additional sealing layer (such as, e.g., the occlusive layer 110) may be attached to a patient to surround the tape-like dressing 100 that has been applied to the patient.

The various to dressing 100 configurations and features described above may apply to all of, or only some of, the components of the dressing 100. For example, as representatively illustrated by the embodiment of FIG. 1, in some embodiments, the occlusive layer 110 and the interface layer 130 may be defined by annular structures configured to be slid onto a foot of the patient, while the decompression layer 120 comprises a tape-like structure that may be wound around the occlusive layer 110 prior to the attachment of the occlusive layer 110.

### Configuration of Exemplary Embodiments

As utilized herein, the terms "approximately," "about," "substantially", and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the disclosure as recited in the appended claims.

It should be noted that the term "exemplary" and variations thereof, as used herein to describe various embodiments, are intended to indicate that such embodiments are possible examples, representations, or illustrations of possible embodiments (and such terms are not intended to connote that such embodiments are necessarily extraordinary or superlative examples).

The term "coupled" and variations thereof, as used herein, means the joining of two members directly or indirectly to one another. Such joining may be stationary (e.g., permanent or fixed) or moveable (e.g., removable or releasable). Such joining may be achieved with the two members coupled directly to each other, with the two members coupled to each other using a separate intervening member and any additional intermediate members coupled with one another, or with the two members coupled to each other using an intervening member that is integrally formed as a single unitary body with one of the two members. If "coupled" or variations thereof are modified by an additional term (e.g., directly coupled), the generic definition of "coupled" provided above is modified by the plain language meaning of the additional term (e.g., "directly coupled" means the joining of two members without any separate intervening member), resulting in a narrower definition than the generic definition of "coupled" provided above. Such coupling may be mechanical, electrical, or fluidic.

The term "or," as used herein, is used in its inclusive sense (and not in its exclusive sense) so that when used to connect a list of elements, the term "or" means one, some, or all of the elements in the list. Conjunctive language such as the phrase "at least one of X, Y, and Z," unless specifically stated otherwise, is understood to convey that an element may be either X, Y, Z; X and Y; X and Z; Y and Z; or X, Y, and Z (i.e., any combination of X, Y, and Z). Thus, such conjunctive language is not generally intended to imply that certain embodiments require at least one of X, at least one of Y, and at least one of Z to each be present, unless otherwise indicated.

References herein to the positions of elements (e.g., "top," "bottom," "above," "below") are merely used to describe the orientation of various elements in the FIGURES. It should be noted that the orientation of various elements may differ according to other exemplary embodiments, and that such variations are intended to be encompassed by the present disclosure.

## Claims

1. An apparatus for applying a lifting force to a tissue site of a patient, the apparatus comprising:
an occlusive layer (110) configured to be sealed to a patient around a tissue site to define a substantially air-tight chamber;
a decompression layer (120) disposed within the chamber defined by the occlusive layer (110) at a location proximate the tissue site, the decompression layer (120) comprising a compressible fabric defining one or more channels therethrough; and
a connector provided along the occlusive layer (110), the connector configured to fluidly couple the chamber to a vacuum source;
wherein, the decompression layer (120) has a centre of stiffness located closer to a surface of the decompression layer (120) facing away from the tissue site than to a surface of the decompression layer (120) facing the tissue site such that upon operation of the vacuum source, the decompression layer (120) is configured to compress in a direction away from the tissue site.

2. The apparatus of claim 1, wherein the occlusive layer (110) extends at least 360° around an extremity defining the tissue site upon being sealed to a patient, and the decompression layer (120) is configured to compress in a radially outwards direction during operation of the vacuum source.

3. The apparatus of claim 1, wherein the occlusive layer (110) extends less than 360° around an extremity defining the tissue site upon being sealed to a patient.

4. The apparatus of claim 1, wherein the decompression layer (120) comprises a macro-mesh material.

5. The apparatus of claim 4, wherein the macro-mesh material includes:
an upper layer (121);
a lower layer(123); and
a plurality of filaments (129) extending between and connecting the upper layer (121) and the lower layer (123).

6. The apparatus of claim 5, wherein the filaments (129) are flexible, such that a distance between the upper layer (121) and the lower layer (123) prior to operation of the vacuum source is greater than a distance between the upper layer (121) and the lower layer (123) during operation of the vacuum source.

7. The apparatus of claim 5, wherein the upper layer (121) extends substantially continuously relative to the lower layer (123).

8. The apparatus of claim 5, wherein the upper layer (121) has a higher stiffness than the lower layer (123).

9. The apparatus of claim 8, wherein the macro-mesh material further comprises a first intermediate layer (122) disposed between the upper layer (121) and the lower layer (123), the first intermediate layer (122) having a higher stiffness than the lower layer (123).

10. The apparatus of claim 9, wherein the first intermediate layer (122) comprises the same material as the upper layer (121).

11. The apparatus of claim 9, wherein the plurality of filaments (129) extend between the first intermediate layer (122) and at least one of the lower layer (123) and the upper layer (121).

12. The apparatus of claim 10, wherein the macro-mesh material further comprises a second intermediate layer disposed between the upper layer (121) and the first intermediate layer (122), the second intermediate layer having a lower stiffness than the first intermediate layer (122).

13. The apparatus of claim 10, wherein the macro-mesh material further comprises a second intermediate layer disposed between the lower layer (123) and the first intermediate layer (122), the second intermediate layer having a lower stiffness than the first intermediate layer (122).

14. The apparatus of claim 1, wherein a center of mass of the decompression layer (120) is located at a height along the decompression layer (120) that is closer to the upper surface of the decompression layer (120) than to the lower surface of the decompression layer (120).

15. The apparatus of claim 1, further comprising an interface layer (130) located below the lower surface of the decompression layer (120), the interface layer (130) contacting skin surrounding the tissue site upon sealing of the occlusive layer to the patient.

## Patentansprüche

1. Eine Vorrichtung zum Aufbringen einer Hebekraft auf eine Gewebestelle eines Patienten, die Vorrichtung aufweisend:
eine Okklusivschicht (110), die konfiguriert ist, um an einem Patienten um eine Gewebestelle herum versiegelt zu werden, um eine im Wesentlichen luftdichte Kammer auszubilden;
eine Dekompressionsschicht (120), die innerhalb der Kammer, die durch die Okklusivschicht (110) definiert ist, an einer Stelle nahe der Gewebestelle angeordnet ist, die Dekompressionsschicht (120) aufweisend einen komprimierbaren Stoff, der einen oder mehrere Kanäle dort hindurch definiert; und
einen Verbinder, der entlang der Okklusivschicht (110) bereitgestellt ist; wobei der Verbinder konfiguriert ist, um die Kammer mit einer Vakuumquelle fluidisch zu koppeln;
wobei die Dekompressionsschicht (120) ein Steifigkeitszentrum aufweist, das sich näher an einer Oberfläche der Dekompressionsschicht (120), die von der Gewebestelle abgewandt ist, als zu einer Oberfläche der Dekompressionsschicht (120), die der Gewebestelle zugewandt ist, befindet, derart, dass bei Betrieb der Vakuumquelle die Dekompressionsschicht (120) konfiguriert ist, um in einer Richtung weg von der Gewebestelle komprimiert zu werden.

2. Die Vorrichtung nach Anspruch 1, wobei sich die Okklusionsschicht (110) um mindestens 360° um eine Extremität herum erstreckt, die die Gewebestelle definiert, wenn sie an einem Patienten versiegelt wird, und die Dekompressionsschicht (120) konfiguriert ist, um in einer radial nach außen gerichteten Richtung während des Betriebs der Vakuumquelle komprimiert zu werden.

3. Die Vorrichtung nach Anspruch 1, wobei sich die Okklusivschicht (110) um weniger als 360° um eine Extremität herum erstreckt, die die Gewebestelle definiert, wenn sie an einem Patienten versiegelt wird.

4. Die Vorrichtung nach Anspruch 1, wobei die Dekompressionsschicht (120) ein Makro-Mesh-Material aufweist.

5. Die Vorrichtung nach Anspruch 4, wobei das Makro-Mesh-Material einschließt:
eine obere Schicht (121);
eine untere Schicht(123); und
eine Vielzahl von Fäden (129), die sich zwischen der oberen Schicht (121) und der unteren Schicht (123) erstrecken und diese verbinden.

6. Die Vorrichtung nach Anspruch 5, wobei die Fäden (129) flexibel sind, derart, dass ein Abstand zwischen der oberen Schicht (121) und der unteren Schicht (123) vor dem Betrieb der Vakuumquelle größer als ein Abstand zwischen der oberen Schicht (121) und der unteren Schicht (123) während des Betriebs der Vakuumquelle ist.

7. Die Vorrichtung nach Anspruch 5, wobei sich die obere Schicht (121) im Wesentlichen kontinuierlich relativ zu der unteren Schicht (123) erstreckt.

8. Die Vorrichtung nach Anspruch 5, wobei die obere Schicht (121) eine höhere Steifigkeit als die untere Schicht (123) aufweist.

9. Die Vorrichtung nach Anspruch 8, wobei das Makro-Mesh-Material ferner eine erste Zwischenschicht (122) aufweist, die zwischen der oberen Schicht (121) und der unteren Schicht (123) angeordnet ist, wobei die erste Zwischenschicht (122) eine höhere Steifigkeit als die untere Schicht (123) aufweist.

10. Die Vorrichtung nach Anspruch 9, wobei die erste Zwischenschicht (122) das gleiche Material wie die obere Schicht (121) aufweist.

11. Die Vorrichtung nach Anspruch 9, wobei sich die Vielzahl von Fäden (129) zwischen der ersten Zwischenschicht (122) und mindestens einer der unteren Schicht (123) und der oberen Schicht (121) erstreckt.

12. Die Vorrichtung nach Anspruch 10, wobei das Makro-Mesh-Material ferner eine zweite Zwischenschicht aufweist, die zwischen der oberen Schicht (121) und der ersten Zwischenschicht (122) angeordnet ist, wobei die zweite Zwischenschicht eine geringere Steifigkeit als die erste Zwischenschicht (122) aufweist.

13. Die Vorrichtung nach Anspruch 10, wobei das Makro-Mesh-Material ferner eine zweite Zwischenschicht aufweist, die zwischen der unteren Schicht (123) und der ersten Zwischenschicht (122) angeordnet ist, wobei die zweite Zwischenschicht eine geringere Steifigkeit als die erste Zwischenschicht (122) aufweist.

14. Die Vorrichtung nach Anspruch 1, wobei sich ein Massenschwerpunkt der Dekompressionsschicht (120) in einer Höhe entlang der Dekompressionsschicht (120) befindet, die näher an der oberen Oberfläche der Dekompressionsschicht (120) als an der unteren Oberfläche der Dekompressionsschicht (120) ist.

15. Die Vorrichtung nach Anspruch 1, ferner aufweisend eine Grenzflächenschicht (130), die sich unter der unteren Oberfläche der Dekompressionsschicht (120) befindet, wobei die Grenzflächenschicht (130) die Haut, die die Gewebestelle umgibt, berührt, wenn die Okklusionsschicht an dem Patienten versiegelt wird.

## Revendications

1. Appareil destiné à appliquer une force d'élévation sur un site tissulaire d'un patient, l'appareil comprenant :
une couche occlusive (110) conçue pour être scellée à un patient autour d'un site tissulaire afin de définir une chambre sensiblement étanche à l'air ;
une couche de décompression (120) disposée dans la chambre définie par la couche occlusive (110) à un endroit proche du site tissulaire, la couche de décompression (120) comprenant un tissu compressible définissant un ou plusieurs canaux à travers celui-ci ; et
un connecteur prévu le long de la couche occlusive (110) ; le connecteur est conçu pour accoupler fluidiquement la chambre à une source de vide ;
dans lequel la couche de décompression (120) a un centre de rigidité situé plus près d'une surface de la couche de décompression (120) orientée à l'opposé du site tissulaire que d'une surface de la couche de décompression (120) orientée vers le site tissulaire, de sorte que, lors du fonctionnement de la source de vide, la couche de décompression (120) est conçue pour se comprimer dans une direction opposée au site tissulaire.

2. Appareil selon la revendication 1, dans lequel la couche occlusive (110) s'étend au moins à 360 ° autour d'une extrémité définissant le site tissulaire après avoir été scellée sur un patient, et la couche de décompression (120) est conçue pour se comprimer dans une direction radiale vers l'extérieur pendant le fonctionnement de la source de vide.

3. Appareil selon la revendication 1, dans lequel la couche occlusive (110) s'étend sur moins de 360 ° autour d'une extrémité définissant le site tissulaire après avoir été scellée sur un patient.

4. Appareil selon la revendication 1, dans lequel la couche de décompression (120) comprend un matériau à macro-maille.

5. Appareil selon la revendication 4, dans lequel le matériau à macro-maille comporte :
une couche supérieure (121) ;
une couche inférieure (123) ; et
une pluralité de filaments (129) s'étendant entre et reliant la couche supérieure (121) et la couche inférieure (123).

6. Appareil selon la revendication 5, dans lequel les filaments (129) sont flexibles, de sorte qu'une distance entre la couche supérieure (121) et la couche inférieure (123) avant le fonctionnement de la source de vide est plus grande qu'une distance entre la couche supérieure (121) et la couche inférieure (123) pendant le fonctionnement de la source de vide.

7. Appareil selon la revendication 5, dans lequel la couche supérieure (121) s'étend de manière sensiblement continue par rapport à la couche inférieure (123).

8. Appareil selon la revendication 5, dans lequel la couche supérieure (121) a une rigidité plus élevée que la couche inférieure (123).

9. Appareil selon la revendication 8, dans lequel le matériau à macromaille comprend en outre une première couche intermédiaire (122) disposée entre la couche supérieure (121) et la couche inférieure (123), la première couche intermédiaire (122) ayant une rigidité supérieure à celle de la couche inférieure (123).

10. Appareil selon la revendication 9, dans lequel la première couche intermédiaire (122) comprend le même matériau que la couche supérieure (121).

11. Appareil selon la revendication 9, dans lequel la pluralité de filaments (129) s'étend entre la première couche intermédiaire (122) et au moins l'une des couches inférieure (123) et supérieure (121).

12. Appareil selon la revendication 10, dans lequel le matériau à macromaille comprend en outre une seconde couche intermédiaire disposée entre la couche supérieure (121) et la première couche intermédiaire (122), la seconde couche intermédiaire ayant une rigidité inférieure à celle de la première couche intermédiaire (122).

13. Appareil selon la revendication 10, dans lequel le matériau à macromaille comprend en outre une seconde couche intermédiaire disposée entre la couche inférieure (123) et la première couche intermédiaire (122), la seconde couche intermédiaire ayant une rigidité inférieure à celle de la première couche intermédiaire (122).

14. Appareil selon la revendication 1, dans lequel un centre de masse de la couche de décompression (120) est situé à une hauteur le long de la couche de décompression (120) qui est plus proche de la surface supérieure de la couche de décompression (120) que de la surface inférieure de la couche de décompression (120).

15. Appareil selon la revendication 1, comprenant en outre une couche d'interface (130) située sous la surface inférieure de la couche de décompression (120), la couche d'interface (130) entrant en contact avec la peau entourant le site tissulaire lors du scellement de la couche occlusive sur le patient.
